# FASCICULE DE BREVET EUROPEEN

(11) **EP 3 256 037 B1**
(45) Date de publication et mention de la délivrance du brevet: **15.02.2023**
(21) Numéro de dépôt: 15817090.2
(22) Date de dépôt: 02.12.2015
(51) Int. Cl.: A61B 5/00, A61B 5/026, A61B 5/103, A61B 5/02

(54) **DISPOSITIF, SYSTÈME ET PROCÉDÉ D'ÉVALUATION DE LA MICROCIRCULATION SANGUINE D'UN TISSU**
VORRICHTUNG, SYSTEM UND VERFAHREN ZUR BEURTEILUNG DER MIKROZIRKULATION VON BLUT IN EINEM GEWEBE
DEVICE, SYSTEM AND METHOD FOR EVALUATING THE MICROCIRCULATION OF BLOOD IN A TISSUE

(30) Priorité: 02.12.2014 FR 1461821
(43) Date de publication de la demande: 20.12.2017
(73) Titulaire: société DICARTECH, 07100 Annonay (FR)
(72) Inventeur: JACQUET-LAGREZE, Matthias, 69007 Lyon (FR)
(74) Mandataire: IP Trust
(86) Numéro de dépôt international: PCT/EP2015/078433
(87) Numéro de publication internationale: WO 2016/087556

(56) Documents cités:
- US-A1- 2004 249 290
- US-A1- 2006 234 383
- US-A1- 2009 143 655
- US-A1- 2012 220 878
- US-A1- 2014 081 161
- US-B1- 6 631 288

## Description

### DOMAINE TECHNIQUE DE L'INVENTION

La présente invention concerne le domaine de l'évaluation de la microcirculation sanguine dans les tissus, en particulier pour des muqueuses, de préférence intra-buccale comme par exemple la muqueuse jugale (de la joue) ou gingivale (de la gencive) mais également d'autres muqueuses comme la conjonctivale (de la paupière), ou pour la peau. Le tissu étudié est de préférence un tissu facilement accessible sur le patient, même lorsqu'il est inconscient. L'invention est particulièrement adaptée pour évaluer la microcirculation chez les patients en réanimation, par exemple atteints d'insuffisance circulatoire aiguë, notamment au cours d'un choc septique. En effet, le choc septique est une défaillance circulatoire aiguë, entraînant des désordres hémodynamiques, métaboliques et viscéraux, généralement dans un contexte de sepsis (syndrome d'infection générale et grave de l'organisme par des germes infectieux). La présente invention trouve en outre son application dans le suivi hémodynamique et thérapeutique des insuffisances circulatoires (choc septique, cardiogénique, hypovolémique ou obstructif) ou dans les situations à risque pour le suivi hémodynamique, comme par exemple lors d'une anesthésie pour une chirurgie à haut risque. De plus, la présente invention trouve des applications dans toutes les situations Cliniques où l'altération microcirculatoire est importante, comme par exemple dans les maladies métaboliques (diabètes, HTA, hypercholestérolémie) ou les vascularites. L'invention pourrait avoir un rôle dans le suivi thérapeutique ou l'évaluation pronostique de ces maladies. Elle permet aussi l'évaluation de l'état d'hydratation (notamment en pédiatrie). En chirurgie, elle pourrait permettre d'évaluer la viabilité des greffons et l'efficacité des anastomoses (chirurgie de lambeaux, greffes de peaux, greffe d'organe). L'invention peut également servir dans l'évaluation des problèmes ischémiques, notamment mésentériques (pour évaluer la viabilité de la séreuse digestive et aider la prise en charge thérapeutique) ou dans les ischémies de membres ou les artériopathies évoluées.

### ARRIERE-PLAN TECHNOLOGIQUE DE L'INVENTION

Un problème dans le domaine de la surveillance des malades, notamment en réanimation, concerne les insuffisances circulatoires. Les insuffisances circulatoires peuvent être définies comme une carence tissulaire aiguë en oxygène. Tous les mécanismes de choc (hypovolémique, obstructif ou distributif) s'accompagnent d'une hypoperfusion (perfusion insuffisante) tissulaire qui résulte de la chute du débit cardiaque par dysfonction de la pompe cardiaque ou lié à une hypovolémie, d'une vasodilatation artério-veineuse ou de l'association de ces trois mécanismes. La persistance de l'ischémie tissulaire conduit à l'apparition de lésions cellulaires hypoxiques et s'accompagne fréquemment d'une réaction inflammatoire systémique susceptible d'entraîner ou d'aggraver des lésions viscérales. Cette hypoperfusion sanguine peut provoquer des marbrures qui sont des traces sur la peau, comparables aux veines du marbre. L'observation d'un tel symptôme fournit donc une indication d'insuffisance circulatoire.

En général, ce problème d'insuffisance circulatoire peut être évalué selon une approche macrocirculatoire ou une approche microcirculatoire. L'approche macrocirculatoire est basée sur la mesure de paramètres tels que la pression artérielle, le débit cardiaque, la fréquence cardiaque. En effet, la macrocirculation concerne les paramètres macroscopiques de la circulation, c'est à dire la pression artérielle, le débit cardiaque, la pression veineuse centrale, la fréquence cardiaque, etc. Les mesures généralement sont faites sur le coeur ou les gros vaisseaux. L'approche microcirculatoire concerne la microcirculation qui commence au niveau des artérioles (généralement de diamètre inférieur à 100 micromètres) et continue avec les capillaires (entre 7 et 20 micromètres pour les plus petits) où se font les échanges avec les cellules. Son évaluation se fait sur les organes, les muqueuses ou la peau. L'approche microcirculatoire repose donc sur l'examen physique du patient, notamment en évaluant l'extension des marbrures ou en évaluant le TRC : temps de recoloration cutané (ou capillaire). Ce terme de TRC a été défini pour la première fois en 1947 comme étant le temps que prend le lit capillaire pour reprendre sa couleur avoir été blanchit par une pression. Le TRC est évalué en général par le clinicien qui applique une pression forte avec l'index ou le pouce sur la peau du patient (classiquement sur le doigt ou sur le thorax), puis enlève rapidement la pression et mesure le temps que met la peau à récupérer sa couleur initiale, soit en comptant, soit avec un chronomètre. Cette technique peut aussi être utilisée sur les muqueuses mais sa mise en oeuvre est alors plus complexe. Il est établi que plus le TRC est long plus l'altération microcirculatoire est importante.

La dissociation entre la macrocirculation et la microcirculation s'explique par le shunt microcirculatoire : de gros capillaires vont être le siège d'une augmentation du débit au détriment de tout un réseau d'autres capillaires, ce qui aboutit à une diminution de la perfusion des tissus sans variation du débit cardiaque. Dans les insuffisances circulatoires aiguës, comme par exemple le sepsis, il est établi que la microcirculation et la macrocirculation sont dissociées et la surveillance des patients ne peut donc pas se baser que sur l'évaluation macrocirculatoire.

Ce problème général s'accompagne du problème de la difficulté de l'évaluation de la microcirculation. En particulier, l'évaluation clinique du TRC est soumise à une grande imprécision, notamment par le fait que la pression, le temps de compression cutané, la lumière ambiante, ou encore la température de la peau sont autant de paramètres qui peuvent varier et influencer le TRC.

Ainsi, il a été développé dans l'art antérieur des méthodes visant à évaluer de manière plus fiable la microcirculation. Par exemple, il est connu dans l'art antérieur, notamment de la demande WO2009/053920A1 un appareil de mesure du temps de recoloration cutané (TRC) de manière digitalisée, pour évaluer l'état d'hydratation. Cet appareil fonctionne typiquement en appliquant une pression contrôlée sur un membre (le pied) et en utilisant une lumière polarisée éclairant la peau et un capteur qui transforme le signal réfléchit sous forme d'image. Ce type de solution présente également l'inconvénient d'être coûteux, notamment à cause de l'utilisation de lumière polarisée et de capteurs correspondants, mais aussi à cause des traitements de données nécessaires. De plus, cette solution présente les inconvénients d'être trop encombrant et, par conséquent, de ne pas permettre une évaluation de la microcirculation sur diverses surfaces cutanées inaccessibles à un tel appareil. De même, cet appareil ne permet pas de faire des mesures sur toute surface cutanée du corps, du fait de la nécessité d'une contrepression (le segment de membre avec la surface de peau à analyser est coincé entre les 2 parties de l'appareil pour maintenir une pression contrôlée). Enfin, cet appareil est fait pour évaluer l'hydratation et permet seulement des mesures cutanées sans tenir compte des variations du TRC liées à la température (en particulier parce qu'il est conçu pour évaluer l'hydratation des enfants ayant une diahrée et ne présentant que peu ou pas de fièvre contrairement au sepsis).

D'autre part, il a été développé dans l'art antérieur des méthodes visant à pallier aux problèmes des techniques de mesure du TRC. Par exemple, il est connu des techniques de vidéomicroscopie permettant une évaluation de la microcirculation par une imagerie des microvaisseaux de l'ordre de 20 micromètres. Cependant, ces techniques présentent les inconvénients d'être coûteuses et nécessiter une interprétation par un expert, avec des temps d'analyse longs, des risques d'artefact de compression et l'absence de possibilité d'une mesure continue. Il est également connu de nombreuses sondes de type laser Doppler pour évaluer la microcirculation à partir d'un signal pulsatile, à différents endroits (comme par exemple le tube digestif, la bouche, les organes génitaux, la surface cutané, une plaie, etc.)

Ces sondes sont généralement basées ou basées sur le principe de la photopléthysmographie (PPG) ou la débitmétrie laser Doppler (LDF pour l'anglais « Laser Doppler Flowmetry »). Le principe de la PPG utilise la réflexion cutanée de la lumière infrarouge au niveau des plexus veineux intradermique. La débitmétrie (ou vélocimétrie) Doppler au laser (LDF) est utilisée dans la recherche de l'hémodynamique comme une technique pour quantifier partiellement le flux sanguin dans les tissus humains telles que la peau. Le faisceau émis par un laser de faible puissance (en général une diode laser) pénètre dans la peau pour être suffisamment dispersé avec un décalage de fréquence Doppler par les globules rouges du sang. Ces techniques ont également l'inconvénient d'être coûteuses. De plus, ces techniques utilisent une mesure à partir d'un signal pulsatile alors que la microcirculation ne l'est pas, ce qui apparente ces techniques à une évaluation de la perfusion en proche amont de la microcirculation. D'autre part, ces techniques présentent des risques d'artefact de compression pour maintenir en contact la sonde avec la zone mesurée.

Les documents US 2004/249290 A1, US 2009/143655 A1 et US 6,631,288 B1 décrivent des dispositifs optiques d'évaluation de la microcirculation aptes à comprimer un tissu.

Dans ce contexte, il est intéressant de fournir une solution peu coûteuse et fiable d'évaluation de la microcirculation.

### DESCRIPTION GENERALE DE L'INVENTION

Un premier but de la présente invention est de pallier au moins un des inconvénients de l'art antérieur en proposant un dispositif d'évaluation de la microcirculation, en particulier peu coûteux et/ou fiable et/ou utilisable sur des portions du corps diverses, même difficilement accessibles.

Ce but est atteint par un dispositif d'évaluation de la microcirculation sanguine d'un tissu humain ou animal, en particulier une muqueuse ou la peau, caractérisé en ce qu'il comporte un boitier comprenant une extrémité ouverte, destinée à être placée au contact dudit tissu et délimiter ainsi une surface d'étude dudit tissu, le dispositif comprenant :
- des moyens d'émission de lumière vers la surface d'étude,
- des moyens d'acquisition d'images de la surface d'étude,
- des moyens de compression d'au moins une zone déterminée à l'intérieur de la surface d'étude, lesdits moyens de compression étant calibrés pour appliquer une pression contrôlée sur ladite zone et rétractables en dehors du champ d'acquisition d'images pour laisser ladite zone de compression et/ou ladite surface d'étude, accessible(s), de préférence complètement accessible(s), à ladite lumière et/ou aux moyens d'acquisition d'images,
- des moyens de contrôle contrôlant l'application de ladite pression contrôlée sur ladite zone pendant un premier temps déterminé, puis le retrait de ladite pression contrôlée et l'acquisition d'image sur ladite surface d'étude exposée à ladite lumière pendant un second temps déterminé, des moyens de traitement de données utilisant les images acquises pour calculer au moins un paramètre relatif à la décoloration et/ou la recoloration dudit tissu.

Selon une autre particularité, au moins une interface homme-machine fournit à un utilisateur du dispositif ledit paramètre relatif à la décoloration et/ou la recoloration dudit tissu.

Selon une autre particularité, ledit paramètre est un temps de recoloration dudit tissu.

Selon une autre particularité, le boitier est muni à son extrémité ouverte d'une jupe délimitant ladite surface d'étude et flexible pour limiter les variations de pression dues à l'application du dispositif contre ledit tissu par un utilisateur.

Selon une autre particularité, ladite jupe est interchangeable, de type matériel à usage unique désinfecté ou stérile.

Selon une autre particularité, les moyens de compression comportent au moins un piston dont l'extrémité destinée à être en contact avec le tissu est interchangeable, de type matériel à usage unique désinfecté ou stérile.

Selon une autre particularité, lesdits moyens de compression sont calibrés par le fait qu'ils comportent au moins un piston monté sur un ressort de compression calibré.

Selon une autre particularité, lesdits moyens de compression sont montés sur un ressort de rappel actionné à la fin dudit premier temps déterminé pour ramener ledit piston et annuler ladite pression contrôlée sur ladite zone.

Selon une autre particularité, lesdits moyens de compression comportent au moins un piston actionné par des moyens de poussée calibrés grâce à un manomètre ou un capteur de pression, par exemple piézoélectrique.

Selon une autre particularité, lesdits moyens de compression comportent des moyens de contrepression permettant de limiter les variations de pression dues à l'application du dispositif contre ledit tissu par un utilisateur.

Selon une autre particularité, lesdits moyens de compression comportent plusieurs pistons appliquant la même pression contrôlée sur plusieurs zones dans ladite surface d'étude.

Selon une autre particularité, lesdits moyens de compression comportent un seul piston munis de plusieurs saillies appliquant la même pression contrôlée sur plusieurs zones dans ladite surface d'étude.

Selon une autre particularité, lesdits moyens de compression comportent plusieurs pistons appliquant des pressions contrôlées différentes sur plusieurs zones dans ladite surface d'étude.

Selon une autre particularité, les moyens de traitement de données calculent au moins un paramètre relatif à la décoloration et/ou la recoloration dudit tissu sur chacune desdites zones et comparent ce paramètre calculé entre lesdites zones (10) pour calculer au moins un paramètre relatif à l'homogénéité entre les paramètres calculés sur les différentes zones.

Selon une autre particularité, le dispositif comporte, à l'extrémité ouverte, au moins un capteur de contact avec ledit tissu pour déclencher la mesure des premier et second temps déterminés.

Selon une autre particularité, le dispositif est dimensionné, au moins au niveau de l'extrémité ouverte, pour une application de pression calibrée sur une muqueuse intra-buccale, gingivale ou conjonctivale.

Selon une autre particularité, le dispositif comporte des moyens de chauffage de ladite surface d'étude.

Selon une autre particularité, lesdits moyens de contrôle sont intégrés dans ledit boitier.

Selon une autre particularité, lesdits moyens de traitement de données sont intégrés dans ledit boitier.

Un autre but de la présente invention est de pallier au moins un des inconvénients de l'art antérieur en proposant un système d'évaluation de la microcirculation, en particulier peu coûteux et/ou fiable et/ou utilisable sur des portions du corps diverses, même difficilement accessibles.

Ce but est atteint par un système d'évaluation de la microcirculation sanguine d'un tissu humain ou animal, en particulier une muqueuse ou la peau, caractérisé en ce qu'il comporte au moins un dispositif selon certains modes de réalisation de l'invention, coopérant avec au moins un dispositif informatique comprenant des moyens de traitement de données calculant ledit paramètre relatif à la décoloration et/ou la recoloration dudit tissu.

Selon une autre particularité, ledit dispositif informatique comporte une interface homme-machine fournissant à un utilisateur du dispositif ledit paramètre relatif à la décoloration et/ou la recoloration dudit tissu.

Selon une autre particularité, ledit dispositif informatique pilote des moyens de contrôle du dispositif et utilise un premier temps déterminé et un second temps déterminé, pour contrôler l'application de ladite pression contrôlée sur ladite zone pendant un premier temps déterminé, puis le retrait de ladite pression contrôlée et l'acquisition d'image sur ladite surface d'étude exposée à ladite lumière pendant un second temps déterminé.

Un autre but de la présente invention est de pallier au moins un des inconvénients de l'art antérieur en proposant un procédé d'évaluation de la microcirculation, en particulier peu coûteux et/ou fiable et/ou utilisable sur des portions du corps diverses, même difficilement accessibles.

Ce but est atteint par un procédé d"utilisation d'un dispositif ou d'un système d'évaluation de la microcirculation sanguine d'un tissu humain ou animal, en particulier une muqueuse ou la peau, caractérisé en ce qu'il comporte les étapes suivantes :
- Application dudit dispositif sur ledit tissu ;
- Application d'une pression calibrée sur ledit tissu pendant un premier temps déterminé, par les moyens de compression situés à l'extrémité ouverte dudit dispositif ;
- Illumination dudit tissu par une lumière à l'extrémité ouverte dudit dispositif et acquisition d'images dudit tissu par ledit dispositif, pendant un second temps déterminé ;
- Retrait des moyens de compression en dehors du champ d'acquisition d'images ;
- Calcul d'au moins un paramètre relatif à la décoloration et/ou la recoloration dudit tissu suite au retrait de ladite pression calibrée.

Selon une autre particularité, le procédé comporte une analyse des images acquises par le dispositif, cette analyse comprenant les étapes suivantes :
- mesure d'une coloration de référence du tissu avant application de ladite pression calibrée ;
- calcul de la différence de la coloration du tissu après application de ladite pression calibrée par rapport à la coloration de référence, à plusieurs temps successifs après le retrait de ladite pression calibrée ;
- détermination d'un temps de recoloration, par mesure du temps auquel ladite différence de coloration redevient inférieure à un seuil déterminé.

Selon une autre particularité, le procédé comporte un affichage d'informations relatives au temps de recoloration.

Selon une autre particularité, le procédé comporte une comparaison de la coloration du tissu à au moins un temps déterminé à proximité du temps de recoloration par rapport à la coloration de référence, pour déterminer une hypercoloration si la coloration à ce temps déterminé est plus intense que la coloration de référence.

Selon une autre particularité, le procédé comporte un affichage d'informations relatives à l'hypercoloration.

Selon une autre particularité, le procédé comporte un calcul d'au moins un paramètre relatif à la décoloration et/ou la recoloration dudit tissu sur une pluralité de zones de ladite surface d'étude et un calcul d'au moins un paramètre relatif à l'homogénéité entre les paramètres calculés sur les différentes zones.

Selon une autre particularité, le procédé est mis en oeuvre par au moins un dispositif selon certains modes de réalisation de l'invention ou au moins un système selon certains modes de réalisation de l'invention.

### DESCRIPTION DES FIGURES ILLUSTRATIVES

D'autres particularités et avantages de la présente invention apparaîtront plus clairement à la lecture de la description ci-après, faite en référence aux dessins annexés, dans lesquels :

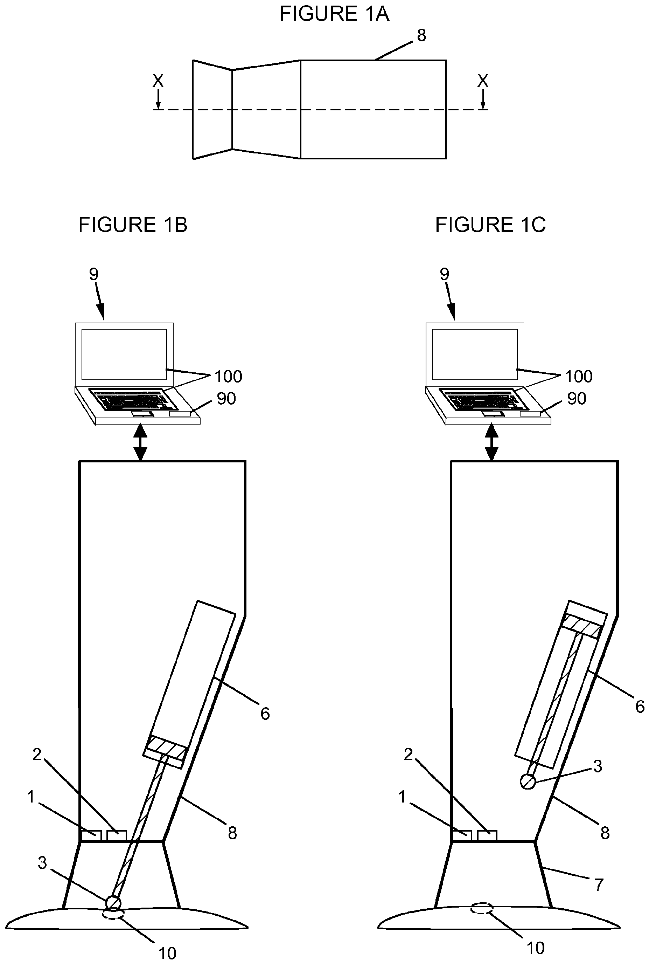

- la figure 1A représente une vue de profil d'un dispositif d'évaluation de la microcirculation selon divers modes de réalisation, les figures 1B et 1C représentent des vues en coupe, selon le plan de coupe X-X de la figure 1A, d'un dispositif d'évaluation de la microcirculation selon divers modes de réalisation, respectivement pendant et après l'application d'une pression calibrée sur un tissu ;

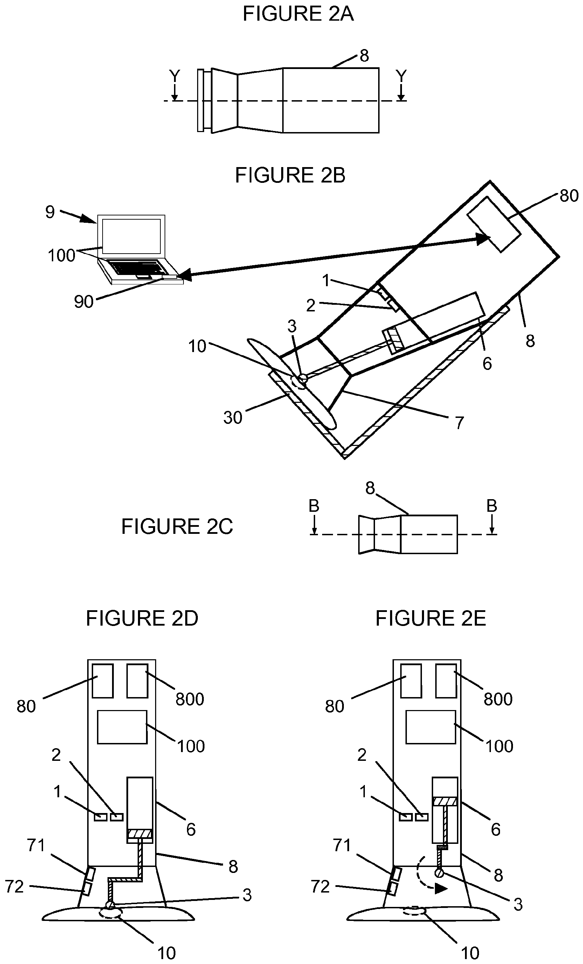

- les figures 2A et 2C représentent une vue de profil de dispositifs d'évaluation de la microcirculation selon divers modes de réalisation, la figure 2B représente une vue en coupe selon le plan de coupe Y-Y de la figure 2A, les figures 2D et 2E représentent des vues en coupe selon le plan de coupe B-B de la figure 1A, d'un dispositif d'évaluation de la microcirculation selon différents modes de réalisation et selon le plan de coupe X-X de la figure 1A, d'un dispositif d'évaluation de la microcirculation selon différents modes de réalisation ;

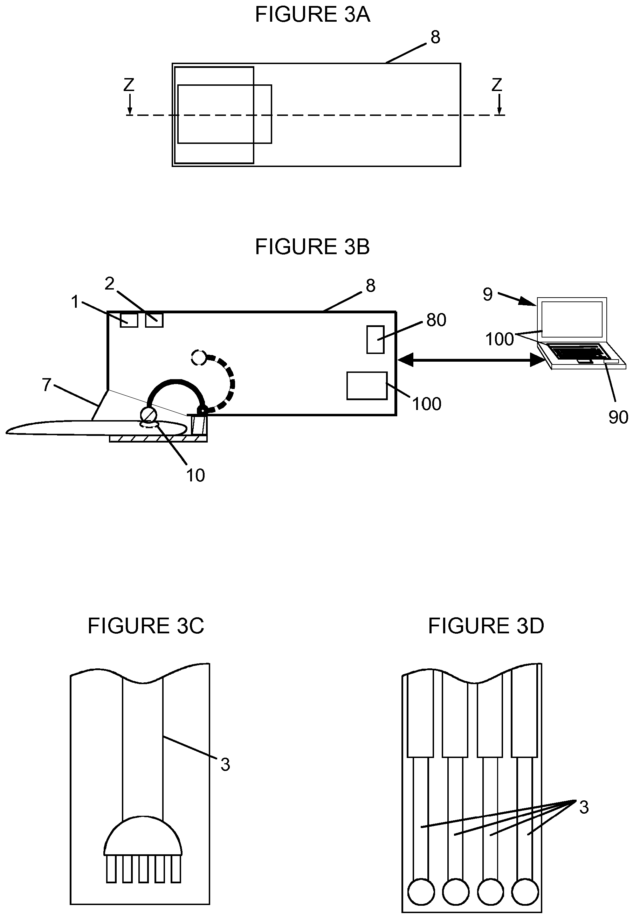

- la figure 3A représente une vue de profil d'un dispositif d'évaluation de la microcirculation selon divers modes de réalisation, et la figure 3B représente une vue en coupe de ce dispositif selon le plan de coupe Z-Z de la figure 3A, les figures 3C et 3D montrent des vues en perspective de l'extrémité de moyens de compression selon différents modes de réalisation ;

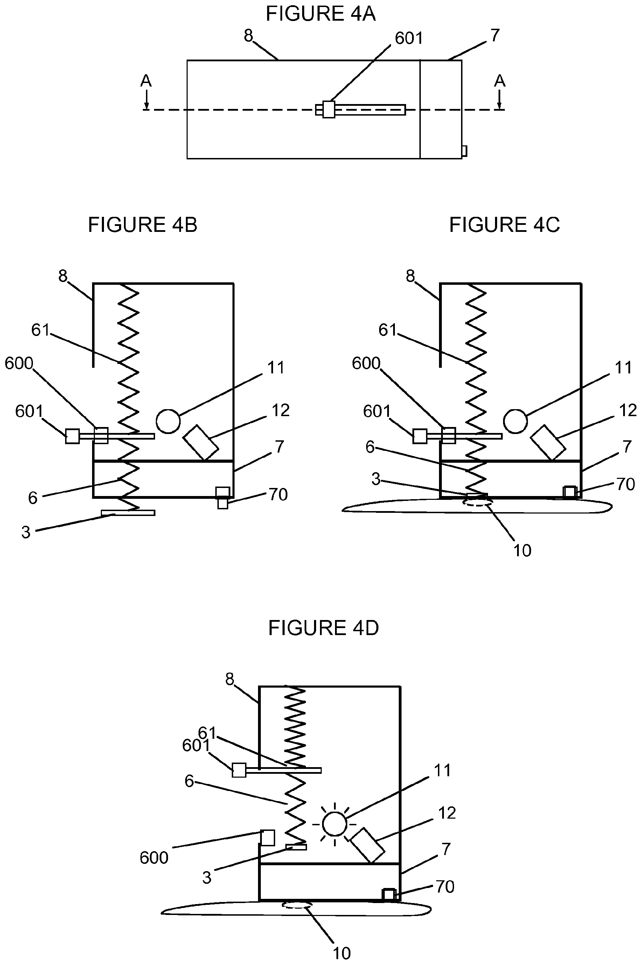

- la figure 4A représente une vue de profil d'un dispositif d'évaluation de la microcirculation selon divers modes de réalisation, les figures 4B, 4C et 4D représentent des vues en coupe, selon le plan de coupe A-A de la figure 4A, d'un dispositif d'évaluation de la microcirculation selon divers modes de réalisation, respectivement après armement du dispositif, pendant l'application d'une pression calibrée sur un tissu et après désarmement du dispositif.

### DESCRIPTION DES MODES DE REALISATION PREFERES DE L'INVENTION

D'une manière générale, la présente invention concerne un dispositif, un système et un procédé d'évaluation de la microcirculation sanguine d'un tissu humain ou animal, en particulier une muqueuse ou la peau. En général, les muqueuses concernées seront les muqueuses buccales, comme les muqueuses gingivales ou jugales, notamment parce qu'elles sont faciles d'accès même chez un patient inconscient (par exemple sous sédatif) et/ou permettent une estimation précise de la microcirculation, mais l'invention peut bien entendu être utilisé sur d'autres muqueuses, par exemple choisies pour leur accès aisé ou leur pertinence dans le pronostic des patients et/ou de leurs traitements.

Dans certains modes de réalisation, dont un exemple illustratif et non limitatif est représenté sur la figure, l'invention concerne un dispositif d'évaluation de la microcirculation sanguine d'un tissu humain ou animal, en particulier une muqueuse ou la peau. Un tel dispositif est caractérisé en ce qu'il comporte un boitier (8) comprenant une extrémité (82) ouverte, destinée à être placée au contact dudit tissu et délimiter ainsi une surface d'étude dudit tissu, le dispositif comprenant :
- des moyens d'émission de lumière (2) vers la surface d'étude,
- des moyens d'acquisition d'images (1) de la surface d'étude,
- des moyens de compression (3, 6) d'au moins une zone déterminée (10) à l'intérieur de la surface d'étude, lesdits moyens de compression (3, 6) étant calibrés pour appliquer une pression contrôlée sur ladite zone (10) et rétractables pour laisser ladite zone de compression, voire même toute ladite surface d'étude entière, accessible à ladite lumière et/ou aux moyens d'acquisition d'images (1), et de préférence complètement accessible (sans perturbation de l'acquisition et/ou de l'illumination),
- des moyens de contrôle (80, 9) contrôlant l'application de ladite pression contrôlée sur ladite zone (10) pendant un premier temps déterminé, puis le retrait de ladite pression contrôlée et l'acquisition d'image sur ladite surface d'étude exposée à ladite lumière pendant un second temps déterminé, des moyens de traitement de données (800, 90) utilisant les images acquises pour calculer au moins un paramètre relatif à la décoloration et/ou la recoloration dudit tissu.

Le boitier est de préférence préhensible à la main. Par exemple, une poignée ergonomique peut être prévue sur le boitier qui peut lui-même également avoir une forme ergonomique, comme par exemple une forme cylindrique ou au moins allongée longitudinalement, par exemple préhensible à une seule main. Une forme par exemple de stylo ou de feutre plus épais qu'un stylo et tenant dans la main peut être particulièrement pratique. Cependant, divers formes de boitier sont envisageables et, dans certains modes de réalisation, on prévoit que le boitier puisse être appliqué sur le tissu sans être tenu par un utilisateur, notamment (mais pas uniquement) lorsque des moyens de contrepression (30) sont prévus sur le dispositif (comme par exemple détaillé plus loin dans la présente demande). Ainsi, le dispositif peut être maintenu au contact du tissu par des moyens de maintien (de diverses formes, comme par exemple un bandage ou une bande collante, un sparadrap, etc.), ou par le fait qu'il comporte des moyens de contrepression de sorte que le tissu soit pris en sandwich dans le dispositif.

Dans certains modes de réalisation, dont un exemple illustratif et non limitatif est représenté sur la figure 2B, lesdits moyens de contrôle (80) sont intégrés dans ledit boitier (8). De tels moyens de contrôle (80) comportent de préférence des contrôleurs pour piloter les divers moyens présents dans le dispositif, via une connectique adaptée. De tels moyens de contrôle (80) peuvent comporter des moyens de mémorisation et de traitement de données pour définir stocker des paramètres tels que les durée d'application de la pression, d'illumination du tissu et d'acquisition d'image et ainsi piloter les divers moyens du dispositif pour en contrôler le fonctionnement. Selon le coût et la complexité souhaitée, il est possible de limiter ces moyens de contrôle à la fonction de pilotage des divers moyens du dispositif d'évaluation et d'utiliser ce dernier sous la supervision d'un dispositif informatique (9), c'est-à-dire un dispositif de contrôle (9) comprenant des moyens informatiques, tel qu'un ordinateur par exemple, comme représenté sur l'exemple illustratif et non limitatif de la figure 2B. Ainsi, Dans certains modes de réalisation, dont un exemple illustratif et non limitatif est représenté sur la figure 2B, ledit dispositif informatique (9) pilote des moyens de contrôle (80) du dispositif pour contrôler l'application de ladite pression contrôlée sur ladite zone (10) pendant un premier temps déterminé, puis le retrait de ladite pression contrôlée et l'acquisition d'image sur ladite surface d'étude exposée à ladite lumière pendant un second temps déterminé.

Dans certains modes de réalisation, dont un exemple illustratif et non limitatif est représenté sur les figures 2C et 2D, lesdits moyens de traitement de données (800) sont intégrés dans ledit boitier (8). Dans ces modes de réalisation, ces moyens de traitement de données pilotent les moyens de contrôle (80) qui pilotent les autres moyens du dispositif, qui peut alors être sensiblement autonome et ne pas nécessiter de dispositif informatique (9) additionnel. Dans certains de ces modes de réalisation, dont un exemple illustratif et non limitatif est représenté sur les figures 2C et 2D, le dispositif muni de tels moyens de traitement de données (800) comporte une interface homme-machine (100) fournissant à un utilisateur du dispositif ledit paramètre relatif à la décoloration et/ou la recoloration dudit tissu. Une telle interface peut bien entendu servir au réglage du dispositif et/ou à l'affichage d'autres paramètres et/ou la sélection de divers protocoles de mesure, par exemple stockés dans une mémoire du dispositif.

En alternative, moins coûteuse pour le dispositif lui-même, mais nécessitant un dispositif informatique (9) additionnel, certains modes de réalisation de l'invention concernent un système d'évaluation de la microcirculation sanguine d'un tissu humain ou animal, en particulier une muqueuse ou la peau, caractérisé en ce qu'il comporte au moins un dispositif selon certains modes de réalisation coopérant avec au moins un dispositif informatique (9) comprenant des moyens de traitement de données (90). Ces moyens (90) de traitement de données calculent ledit paramètre relatif à la décoloration et/ou la recoloration dudit tissu. Dans certains de ces modes de réalisation, dont un exemple illustratif et non limitatif est représenté sur les figures 1B et 1C, ledit dispositif informatique (9) comporte une interface homme-machine (100) fournissant à un utilisateur du dispositif ledit paramètre relatif à la décoloration et/ou la recoloration dudit tissu. Comme mentionné plus haut, une telle interface peut bien entendu servir à diverses fonctions de réglage, calibration, pilotage, sélection de séquences de mesure, etc. D'une manière générale, une telle interface est connue de l'homme de métier et les utilisations qui en découlent n'ont pas à être détaillées. De plus, les figures représentent un écran d'ordinateur et un clavier, mais on comprend qu'un écran tactile est équivalent et qu'en fonction des modes de réalisation choisis, on pourra choisir diverses fonctions à cette interface et divers moyens d'interface (visuel, tactile, sonore, etc.) En effet, il est possible de simplement munir le dispositif d'évaluation fournissant des sons (ou séquences de son) pour indiquer à l'utilisateur quelle action réaliser (appliquer le dispositif, appuyer plus ou moins, retirer le dispositif, etc.) et/ou le résultat de l'évaluation (par exemple un bip court pour une bonne microcirculation et un bip long pour une mauvaise microcirculation, si l'on se contente d'une indication aussi vague).

Dans certains modes de réalisation, les moyens d'émission de lumière comportent au moins une source de lumière. De préférence, on choisit une émission de lumière dans le spectre visible ou infrarouge (ou proche infrarouge). Il est possible de prévoir un spectre polychromatique ou non, comme par exemple une lumière blanche ou monochromatique. Avantageusement, de tels moyens d'émission comportent donc (ou même sont formés par) une source de lumière simple et peu coûteuse.

D'autre part, dans certains modes de réalisation, les moyens d'acquisition d'images comportent (ou même sont formés par) au moins un dispositif vidéo ou photo. En effet, dans de tels modes de réalisation, notamment lorsqu'une source de lumière simple et peu coûteuse est utilisée, l'invention tire avantage des images simples acquises de manière peu coûteuse, comme par exemple à l'aide d'une caméra ou capteur optoélectronique, comme un capteur cMOS (« Complementary Metal Oxide Semiconductor » selon la terminologie anglo-saxonne) par exemple, permettant l'acquisition de séquences vidéo ou d'images en rafales. Divers modes de réalisation tirent donc avantage d'une source de lumière simple et peu coûteuse qui suffit à un capteur de lumière pour mesurer simplement la coloration du tissu avant et après l'application d'une pression calibrée. Cette mesure de la coloration peut simplement concerner une estimation, à partir des images acquises, de la saturation en couleur, notamment une saturation de la teinte magenta, mais peut bien entendu concerner la saturation de diverses teintes. D'autre part, on comprend également que l'invention tire avantage de ces moyens d'émission lumineuse et d'acquisition d'image qui peuvent tous deux être de dimensions très réduite. En effet, une telle source de lumière peut prendre la forme d'une ampoule de petite taille (de l'ordre de la taille de l'extrémité d'un stylo à bille) ou même d'une LED (ou DEL en français pour « diode électroluminescente ») et peut donc ne pas excéder 5 millimètres de diamètre. De même, les moyens d'acquisition d'image peuvent comporter un capteur, par exemple de type cMOS comme détaillé plus haut, dont la taille peut être très restreinte, par exemple de l'ordre de 5 millimètres de diamètre également. D'autre part, les moyens d'acquisition, bien que désignés comme faisant une acquisition d'image, peuvent en fait des capteurs de lumières simples ne captant pas forcément toutes les longueurs d'ondes. Enfin, la pression calibrée peut être appliquée sur le tissu à l'aide d'un bâtonnet ou piston de dimensions restreintes, comme par exemple l'extrémité d'un piston de seringue de 1mL. En général, on choisira d'appliquer une pression calibrée sur une surface comprise entre 25 mm² et 100 mm², de préférence 50mm² (soit sur une surface d'environ 5 millimètres de diamètre, ou 1 centimètre de diamètre), mais il est possible d'utiliser une pression sur une surface plus étendue, notamment (mais pas uniquement) dans divers modes de réalisation détaillés plus loin où l'on étudie également l'hétérogénéité sur plusieurs zones au sein de la surface d'étude. Avantageusement, la combinaison de tels moyens simples et de taille réduite permet d'obtenir un dispositif peu coûteux et de dimensions réduites (au moins au niveau de l'extrémité ouverte où est réalisée la mesure) le rendant facile d'utilisation, mais surtout utilisable sur une grande diversité de tissus, même difficilement accessible. En effet, avec de telles dimensions de la source de lumière, du capteur et du bâtonnet, le dispositif obtenu peut avoir avantageusement une extrémité ouverte dont le diamètre n'excède pas le centimètre ou quelques centimètres seulement (par exemple une taille totale de l'ordre de celle d'un pouce). Ainsi, le dispositif peut aisément être utilisé sur les muqueuses buccales (jugales ou gingivales notamment) ou les muqueuses conjonctivales (en soulevant la paupière par exemple, notamment si le patient est intubé et muni d'appareils rendant difficile l'accès à sa bouche), en plus de n'importe quel autre tissu comme la peau par exemple. Ainsi, dans certains modes de réalisation, dont des exemples illustratifs et non limitatifs sont représentés sur les figures annexées à la présente demande, le dispositif est dimensionné, au moins au niveau de l'extrémité ouverte, pour une application de pression calibrée sur une muqueuse intra-buccale, gingivale ou conjonctivale.

Selon la présente invention, le dispositif utilise des moyens de compression qui applique une pression calibrée sur une zone déterminée du tissu et qui se rétractent en dehors du champ surveillé par les moyens d'acquisition. Ce retrait permet d'étudier précisément la zone comprimée et éventuellement la zone qui l'entoure, dans une surface d'étude donnée. En effet, la plupart des données cliniques indiquent que c'est la recoloration de la zone comprimée qui est un facteur déterminant, car elle révèle des paramètres critiques comme la mortalité, la gravité du choc (septique, hémorragique, etc.) Il est donc utile d'avoir accès directement à la zone qui a été comprimée (et éventuellement celle adjacente), sans perturbation de l'illumination par un objet et sans perturbation de l'acquisition d'image par un matériau interposé entre les moyens d'acquisition et le tissu. En effet, il est connu des dispositifs dans lesquels un matériau transparent est interposé entre les moyens d'acquisition pour appuyer sur le tissu à l'aide de ces derniers, mais cette solution perturbe la mesure par ce matériau qui nécessite d'adapter les moyens d'acquisition et surtout dont la transparence et la stérilité ne sont pas stables puisqu'il vient se souiller au contact du tissu étudié. De plus, comme une hypercoloration se produit parfois lors d'une compression des tissus qui est trop élevée pour l'état physiologique de ces tissus, il peut être utile de comparer les différence de colorations entre la zone qui a été comprimée avec celle à côté. Enfin, par l'utilisation de moyens de compression rétractables, la présente invention permet de faire une évaluation de la recoloration selon diverses techniques. Une première technique consiste à faire l'acquisition de la valeur contrôle de coloration (ou "coloration de référence") avant l'application de la pression, dans la zone qui va être comprimée, ce qui est par exemple avantageux si la lumière n'est pas complètement homogène dans le champ d'acquisition sur toute la surface possible d'étude. Une seconde technique consiste à faire l'acquisition de la valeur contrôle de coloration autour de la zone comprimée, ce qui peut être avantageux en cas d'hypercoloration ou d'instabilité de l'utilisateur avant l'application de la pression. Une troisième technique consiste à ne pas réellement utiliser faire l'acquisition d'une valeur contrôle avant ou autour, mais de s'en tenir à la zone comprimée en ne prenant que la mesure de coloration une fois la zone recolorée, en prenant la valeur de coloration au plateau, par exemple en calculant la dérivée du signal de coloration et en détectant son retour à zéro. Ces techniques sont citées ici à titre illustratif pour montrer les avantages de l'utilisation de moyens de compression rétractables mais elles ne sont pas exhaustives, ni limitatives. D'autre part, l'utilisation de ces moyens de compression rétractables en dehors du champ d'acquisition, en permettant d'étudier précisément la zone comprimée en plus des zones adjacentes dans la surface d'étude, présente l'avantage de permettre l'utilisation de plusieurs zones de compression et d'affiner l'analyse, comme par exemple dans certains modes de réalisation décrits dans la présente demande.

De préférence, comme le dispositif est destiné à être placé au contact du patient (et souvent au contact des muqueuses) on choisit de préférence (et au minimum, en général) d'utiliser des moyens classés comme « semi-critique » et/ou « risque infectieux médian » et/ou « usage unique avec désinfection intermédiaire ». On peut choisir carrément d'utiliser des moyens stériles, mais il semble généralement suffisant d'utiliser un matériel connu pour être défini comme étant de type « matériel à usage unique désinfecté ou stérile ». Ainsi, l'extrémité du bâtonnet ou piston utilisé pour appliquer la pression calibrée sera de préférence interchangeable, de type matériel à usage unique, désinfecté ou stérile. On utilise ici le terme « de type » pour indiquer que la dénomination exacte importe peu et qu'il s'agit avant tout d'obtenir une fonction de protection contre les germes. Dans certains modes de réalisation, dont un exemple illustratif et non limitatif est représenté sur les figures 1A et 1B par exemple, les moyens de compression (3, 6) comportent au moins un piston (3) dont l'extrémité destinée à être en contact avec le tissu est interchangeable, de type matériel à usage unique, désinfecté ou stérile.

Dans certains modes de réalisation, dont un exemple illustratif et non limitatif est représenté sur les figures 1A, 1B, 2A et 2B, le boitier (8) est muni à son extrémité ouverte d'une jupe (7) délimitant ladite surface d'étude. Cette jupe permet de maintenir la surface d'étude parfaitement dans le noir et de la soumettre uniquement à la lumière du dispositif (sans lumière parasite extérieure) pour optimiser l'évaluation de la microcirculation. De plus, une telle jupe est de préférence choisie flexible pour limiter les variations de pression dues à l'application du dispositif contre ledit tissu par un utilisateur. De préférence, dans ces modes de réalisation, ladite jupe (7) est également interchangeable, de type « matériel à usage unique désinfecté ou stérile ».

Concernant les moyens de compression eux-mêmes, l'homme de métier comprendra qu'il est possible diverses structures pour appliquer une pression calibrée et la présente demande détaillent divers modes de réalisation avantageux qui ne doivent pas être interprété de manière limitative. Dans certains modes de réalisation lesdits moyens de compression (3, 6) sont calibrés par le fait qu'ils comportent au moins un piston (3) monté sur un ressort de compression calibré (6). On notera d'ailleurs que les termes piston ou bâtonnet ou autres utilisés dans la présente demande ne doivent pas être considérés de manière limitative et qu'ils désignent éventuellement toute structure, de toute forme, capable d'appliquer une pression, comme par exemple une extrémité de tige ou de bâton ou n'importe quelle structure, comme par exemple une simple plaque. En effet, dans les exemples des figures 4B, 4C et 4D, la pression est appliquée par une plaque montée sur des moyens élastiques (6, 61), désignés ci-après « ressorts » bien qu'il faille en fait les interpréter façon non limitative.

Dans certains modes de réalisation, dont un exemple illustratif et non limitatif est représenté sur les figures 1B et 1C, lesdits moyens de compression (3, 6) comportent au moins un piston (3) actionné par des moyens de poussée (6) calibrés grâce à un manomètre ou un capteur de pression, par exemple piézoélectrique. Divers moyens moteurs sont possibles pour les moyens de poussée et on peut utiliser des moyens utilisant une translation d'un piston ou bâtonnet, mais il est possible d'utiliser une rotation, comme par exemple représenté sur la figure 3B ou une combinaison de translation et rotation comme par exemple représenté sur les figures 2C et 2D. Dans cet exemple des figures 2C et 2D, le piston comporte une extrémité coudée permettant d'appliquer la pression en face du champ de la source de lumière et des moyens d'acquisition d'images. Cet agencement fournit une alternative aux modes de réalisation où le piston n'est pas coudé et doit donc installé de biais par rapport au reste du dispositif pour laisser le champ libre après application de pression, comme par exemple représenté sur les figures 1B et 1C. Cette alternative, plus complexe à cause des deux types de mouvements nécessaire, peut rester avantageuse pour limiter les dimensions du dispositif puisqu'il n'est pas nécessaire de place le piston obliquement. Dans cet exemple de combinaison de mouvements, la translation sert à exercer la poussée pour appliquer la pression calibrée et la rotation sert à pousser l'extrémité du piston en-dehors du champ d'illumination et/ou du champ d'acquisition d'image. On notera également qu'il est possible, avantageusement) de limiter la taille du dispositif et ne mettant pas le piston oblique mais en mettant les moyens lumineux et d'acquisition placés obliquement, comme par représenté sur les figures 4B, 4C et 4D pour mesure la zone. Enfin, dans certains modes de réalisation, dont un exemple illustratif et non limitatif est représenté sur la figure 3B, les moyens de compression sont actionnés en rotation, par exemple grâce à un bâtonnet courbé qui se rétracte dans la largeur du boitier et se déploie jusqu'à la surface d'étude par une rotation autour d'un axe à proximité de l'extrémité ouverte. Un tel agencement permet également que le bâtonnet laisse le champ libre à la lumière et à l'acquisition d'image.

Dans certains modes de réalisation, le boitier (8) comporte une extrémité ouverte (82) dans le prolongement du dispositif (i.e., une extrémité distale ouverte, par exemple dans l'axe longitudinal), comme représenté sur la plupart des figures. Néanmoins, pour pouvoir étudier des surfaces auxquelles l'accès se ferait plus facilement selon un axe non perpendiculaire à la surface d'étude, voire même parallèle à la surface, certains modes de réalisation prévoient une extrémité ouverte (82) au niveau de la paroi latérale du boitier (et non pas la paroi distale). Un exemple illustratif et non limitatif de tels modes de réalisation est représenté sur la figure 3B montrant que les moyens lumineux (2) et d'acquisition d'image (1) sont disposés en face de l'ouverture latérale (82), perpendiculairement à l'axe longitudinal du dispositif. Dans cet exemple de la figure 3B, les moyens de compression sont actionnés en rotation, mais il est possible d'utiliser des moyens actionnés en translation, notamment obliquement, comme dans d'autres modes de réalisation illustrés dans la présente demande. De même, cet exemple illustre un dispositif muni de moyens de contrepression (30) dont l'utilité est détaillée plus loin, mais cet exemple n'est pas limitatif. En particulier, ces moyens de contrepression (30) sont orientés vers l'extrémité distale (fixés avant l'ouverture et s'étendant jusque devant l'ouverture) mais il est possible de les orienter dans l'autre sens, c'est-à-dire vers l'extrémité proximale au lieu de l'extrémité distale ou selon diverses orientations, de sorte que leur fixation sur le boitier soit prévue pour l'utilisation qui en est faite (en fonction du tissu à étudier). De plus, on peut bien entendu se passer de tels moyens de contrepression (30) dans ces modes de réalisation à ouverture latérale.

Dans certains modes de réalisation, notamment parmi les modes de réalisation où lesdits moyens de compression (3, 6) comportent au moins un piston (3) monté sur un ressort de compression calibré (6), lesdits moyens de compression (3, 6) sont montés sur un ressort de rappel (61) actionné à la fin dudit premier temps déterminé pour ramener ledit piston (3) et annuler ladite pression contrôlée sur ladite zone (10). Un exemple illustratif et non limitatif de ces modes de réalisation est représenté sur les figures 4B, 4C et 4D. Dans ces exemples, lesdits moyens de compression (3, 6), montés sur un ressort de rappel (61), doivent être armés par l'utilisateur grâce à des moyens d'armement (600, 601), par exemple tel qu'un curseur (601) accessible sur la paroi du boitier (8) et entrainé par coulissement pour allonger le ressort de rappel (61). Le curseur (601) est alors retenu en position armée par un loquet (600). Un tel loquet (600) permet de relâcher le curseur (601), de préférence automatiquement sous le contrôle des moyens de contrôle (9, 80) lorsque le temps d'application de la pression est écoulé. Ce relâchement avec un tel loquet permet un actionnement rapide, à la fin dudit premier temps déterminé pour ramener ledit piston (3) et annuler ladite pression contrôlée sur ladite zone (10). Dans certains de ces modes de réalisation, il est possible de prévoir des moyens de réglage de la pression, par exemple grâce à plusieurs loquets (600) ou à un loquet de position réglable. En effet, si la course complète du curseur (601) portant le ressort de compression (6) est définie pour que le ressort puisse appliquer une pression déterminée (maximale) lorsque l'extrémité ouverte du dispositif est appliqué sur le tissu, il est possible de régler la position à laquelle on veut arrêter le curseur de sorte que la pression appliquée soit inférieure (à la pression maximale). Des graduations, par exemple avec des valeurs de pressions, pourront d'ailleurs être reportés sur le boitier pour guider l'utilisateur. On notera que, même dans ces dernières variantes réglables, ces modes de réalisation ont l'avantage d'être simples et peu coûteux à fabriquer et à utiliser, tout en fournissant une évaluation fiable de la recoloration. La présente invention permet d'ailleurs, dans la plupart des modes de réalisation, la mise en oeuvre d'un procédé très facile pour l'utilisateur qui n'a qu'à appliquer le dispositif sur le tissu et le laisser lui fournir le résultat.

Dans certains modes de réalisation, dont un exemple illustratif et non limitatif est représenté sur les figures 4B, 4C et 4D, le dispositif comporte, à l'extrémité ouverte, au moins un capteur (70) de contact avec ledit tissu pour déclencher la mesure des premier et second temps déterminés. Un tel capteur de contact peut également être ou comporter un capteur de pression, permettant de vérifier que l'utilisateur n'applique pas lui-même une pression trop importante qui risque d'influencer la pression, de préférence calibrée, appliquée par le dispositif. Un tel capteur est bien entendu particulièrement avantageux dans le cas de l'utilisation d'un ressort de rappel, pour déclencher le décompte du temps d'application et arrêter automatiquement la compression. On notera d'ailleurs que dans les modes de réalisation comprenant un tel ressort de rappel, on utilise de préférence un ressort de compression calibré, mais qu'il est possible de remplacer ces ressorts par tout moyen élastique, de préférence d'élasticité calibrée, et que l'on peut même choisir de remplacer le ressort de compression (6) par un autre moyen, par exemple le capteur de contact (70), de préférence lorsque ce dernier mesure la pression du contact pour permettre d'appliquer une pression correcte sans trop dépendre de l'habilité de l'utilisateur.

Dans certains modes de réalisation, dont un exemple illustratif et non limitatif est représenté sur la figure 2A, lesdits moyens de compression (3, 6) comportent des moyens de contrepression (30) permettant de limiter les variations de pression dues à l'application du dispositif contre ledit tissu par un utilisateur. De tels moyens de contrepression (30) peuvent comporter une plaque, de position stable par rapport aux moyens de compression et destinée à être placée en vis-à-vis des moyens de compression afin de coincer le tissu à étudier entre les moyens de compression et les moyens de contrepression. La position stable des moyens de contrepression permet que la pression calibrée soit appliquée de manière fiable, puisqu'elle n'est du qu'aux moyens de compression pressant le tissu contre les moyens de contrepression. Ainsi, la pression exercée par le dispositif est totalement indépendante de la pression que pourrait exercer maladroitement l'utilisateur. On notera que la position stable des moyens de contrepression par rapport aux moyens de compression peut être réglable pour adapter le dispositif à l'épaisseur des tissus à coincer entre les moyens de compression et les moyens de contrepression. Dans de tels modes de réalisation, la présence de la jupe n'est pas forcément nécessaire mais elle peut néanmoins être conservée pour isoler la surface d'étude du reste des tissus et surtout maintenir la surface d'étude soumise uniquement à la source de lumière du dispositif (aucune lumière ambiante).

Dans certains modes de réalisation, dont un exemple illustratif et non limitatif est représenté sur la figure 3C, lesdits moyens de compression (3, 6) comportent un seul piston (3) munis de plusieurs saillies appliquant la même pression contrôlée sur plusieurs zones (10) dans ladite surface d'étude. De tels modes de réalisation, il est possible de vérifier l'homogénéité de la réaction du tissu à la pression, comme détaillé ci-après. Dans certains modes de réalisation, dont un exemple illustratif et non limitatif est représenté sur la figure 3D, lesdits moyens de compression (3, 6) comportent plusieurs pistons (3) appliquant soit la même pression contrôlée sur plusieurs zones (10) dans ladite surface d'étude, soit des pressions contrôlées différentes sur plusieurs zones (10) dans ladite surface d'étude. L'application de différentes pressions permet éventuellement de vérifier la pertinence du test pour chacune des pressions appliquées. Dans certains de ces modes de réalisation, dont un exemple illustratif et non limitatif est représenté sur la figure, les moyens de traitement de données (800, 90) calculent au moins un paramètre relatif à la décoloration et/ou la recoloration dudit tissu sur chacune desdites zones (10) et calculent au moins un paramètre relatif à l'homogénéité entre les paramètres calculés sur les différentes zones (10). On notera que dans certains modes de réalisation, il est possible de calculer également une homogénéité sur différentes zones, même si une pression a été appliquée sur une seule zone donnée. En effet, il est possible de subdiviser ladite zone en plusieurs cadrans et de calculer les paramètres souhaités pour chacun de ces cadrans, de sorte que l'on obtient une comparaison des cadrans. De préférence, la subdivision entre les cadrans prévoit un espace entre chaque cadran, de façon à éviter un recouvrement entre les cadrans et à éviter des calculs sur des cadrans dont une portion peut présenter les mêmes propriétés que le cadran voisin (notamment à cause des vaisseaux sanguins qui peuvent s'étendre sur une région commune à deux cadrans).

Dans certains modes de réalisation, dont un exemple illustratif et non limitatif est représenté sur les figures 2C et 2D, le dispositif comporte des moyens de chauffage (71) de ladite surface d'étude. Dans certains modes de réalisation, le dispositif comporte des moyens de mesure de température (72) de la surface d'étude ou à proximité de cette dernière, de sorte qu'un facteur correctif puisse être appliqué à l'évaluation de la microcirculation en fonction de la température mesurée. De préférence, une telle mesure de la température est également prévue dans les modes de réalisation où le dispositif comporte des moyens de chauffage, par exemple pour contrôler la nécessité de recourir à ces derniers, décider de la température à appliquer et contrôler la température lors du chauffage. On notera que l'on parle ici de chauffage, mais qu'on entend en fait un réchauffement délicat pour éviter de léser les tissus. De tels moyens de chauffage ou réchauffement sont donc de préférence muni de moyens de diffusion progressive de la chaleur vers le tissu, ou au minimum muni de moyens de protection du tissu contre les brûlures. De tels modes de réalisation sont particulièrement avantageux (mais pas seulement) dans le cas d'une utilisation sur un tissu tel que la peau qui est soumis à des phénomènes de thermorégulation, c'est-à-dire des mécanismes qui permettent de maintenir l'organisme à température constante (constriction des capillaires cutané lors du froid et dilatation à la chaleur). Ainsi, en contrôlant la température, on peut la prendre en compte dans l'évaluation et, idéalement, en régulant la température, on évite que ces phénomènes ne viennent perturber la mesure par le dispositif.

Dans certains modes de réalisation, comme déjà mentionné, au moins une interface homme-machine (100) fournit à un utilisateur du dispositif ledit paramètre relatif à la décoloration et/ou la recoloration dudit tissu. Dans certains modes de réalisation, ledit paramètre est au moins un temps de recoloration dudit tissu ou un indice de recoloration ou de microcirculation déterminé sur la base du temps calculé et de la connaissance des phénomènes circulatoires. En effet, le dispositif selon divers modes de réalisation permet une utilisation aisée sur les patients et permet la mise en oeuvre de divers modes de réalisation d'un procédé d'évaluation de la microcirculation, en fonction des mesures effectuées. Ainsi, dans certains modes de réalisation, l'invention concerne un procédé d'évaluation de la microcirculation sanguine d'un tissu humain ou animal, en particulier une muqueuse ou la peau, caractérisé en ce qu'il comporte les étapes suivantes :
- Application du dispositif sur le tissu;
- Application d'une pression calibrée sur ledit tissu pendant un premier temps déterminé, par des moyens de compression (3, 6) situés à l'extrémité ouverte d'un dispositif d'évaluation de la microcirculation sanguine, appliqué sur ledit tissu ;
- Illumination dudit tissu par une lumière à l'extrémité ouverte dudit dispositif et acquisition d'images dudit tissu par ledit dispositif, pendant un second temps déterminé ;
- Calcul d'au moins un paramètre relatif à la décoloration et/ou la recoloration dudit tissu suite au retrait de ladite pression calibrée.

Avantageusement, le procédé met en oeuvre dans le dispositif une étape de retrait des moyens de compression (3, 6) en dehors du champ d'acquisition d'images. Les avantages de cette étape sont détaillés plus haut en référence au dispositif et n'ont pas besoin d'être rappelés.

Dans certains modes de réalisation, le procédé comporte une analyse des images acquises par le dispositif, cette analyse comprenant les étapes suivantes :
- mesure d'une coloration de référence du tissu avant application de ladite pression calibrée ;
- calcul de la différence de la coloration du tissu après application de ladite pression calibrée par rapport à la coloration de référence, à plusieurs temps successifs après le retrait de ladite pression calibrée ;
- détermination d'un temps de recoloration, par mesure du temps auquel ladite différence de coloration redevient inférieure à un seuil déterminé.

Comme détaillé en référence au dispositif, diverses techniques sont possibles et l'étape de mesure de la coloration de référence n'est pas forcément réalisée sur la zone de compression, voire pas forcément nécessaire si l'on utilise simplement le critère recoloration par la dérivée du signal mesuré. En général, la recoloration des tissus suit une évolution assez linéaire et rapide, de sorte qu'il suffit de mesure le temps de recoloration. Le fait de déterminer quand la coloration est revenue à la normale (ou proche de la normale, avec un seuil de tolérance déterminé) est donc suffisant. Néanmoins, il est bien entendu possible de mesurer plusieurs temps différents, notamment des temps déterminés par référence à une courbe logarithmique si cela s'avère nécessaire. Dans certains modes de réalisation, le procédé comporte un affichage d'informations relatives au temps de recoloration, c'est-à-dire soit le temps lui-même, soit un indice déterminé, comme par exemple un indice d'évaluation de la microcirculation. En effet, le dispositif lui-même ou le dispositif informatique de contrôle (9) peut stocker une table de correspondance entre les données relatives au temps et à la microcirculation, mais l'utilisateur généralement versé dans l'art n'a souvent pas besoin d'un indice et sait baser ses conclusions sur la valeur de temps de recoloration.

Dans les modes de réalisation où la microcirculation est évaluée sur plusieurs zones (10) ou plusieurs cadrans au sein de la zone (10) étudiée, on comprend que le procédé est appliqué de la même manière, mais que l'analyse sera réitérée pour chaque cadran ou zone. De plus, l'analyse permet alors une comparaison des zones ou cadrans entre elles (ou eux) et un calcul d'un facteur d'homogénéité, par exemple tel que la différence entre le temps le plus long et le temps le plus court entre deux zones/cadrans, de préférence divisé par la moyenne des temps des zones/cadrans.

Dans certains modes de réalisation, le procédé comporte une comparaison de la coloration du tissu à au moins un temps déterminé à proximité du temps de recoloration (par exemple le temps auquel la différence de coloration est devenue inférieure au seuil, comme expliqué ci-dessus) par rapport à la coloration de référence, pour déterminer une hypercoloration si la coloration à ce temps déterminé est plus intense que la coloration de référence. En effet, il est souvent observé que les tissus des patients de certaines affections peuvent présenter une hyperhémie, c'est-à-dire une augmentation de la coloration par rapport à l'état basal, témoignant généralement d'une altération endothéliale (de la paroi des vaisseaux). Ainsi, grâce à l'interface homme-machine, dans certains modes de réalisation, le procédé comporte un affichage d'informations relatives à l'hypercoloration (ou l'hyperhémie).

On comprend de la description qui précède pour le dispositif et le système, que selon divers modes de réalisation, le procédé est mis en oeuvre par au moins un dispositif selon certains modes de réalisation de l'invention ou au moins un système selon certains modes de réalisation l'invention. L'homme de métier comprendra donc toutes les implications des divers modes de réalisation de dispositif et de système en termes de procédé.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Dispositif d'évaluation de la microcirculation sanguine d'un tissu humain ou animal, en particulier une muqueuse ou la peau, comportant :
- un boitier (8) comprenant une extrémité (82) ouverte, destinée à être placée au contact dudit tissu et délimiter ainsi une surface d'étude dudit tissu, le dispositif comprenant :
- des moyens d'émission de lumière (2) vers la surface d'étude,
- des moyens d'acquisition d'images (1) de la surface d'étude,
- des moyens de compression (3, 6) d'au moins une zone déterminée (10) à l'intérieur de la surface d'étude, lesdits moyens de compression (3, 6) étant calibrés pour appliquer une pression contrôlée sur ladite zone (10) et rétractables en dehors du champ d'acquisition d'images pour laisser ladite surface de compression et/ou d'étude accessible à ladite lumière et aux moyens d'acquisition d'images,
- des moyens de contrôle (80, 9) contrôlant l'application de ladite pression contrôlée sur ladite zone (10) pendant un premier temps déterminé, puis le retrait de ladite pression contrôlée et l'acquisition d'image sur ladite surface d'étude exposée à ladite lumière pendant un second temps déterminé,
- des moyens de traitement de données (800, 90) utilisant les images acquises pour calculer au moins un paramètre relatif à la décoloration et/ou la recoloration dudit tissu.

2. Dispositif selon la revendication 1, **caractérisé en ce que** ledit paramètre est un temps de recoloration dudit tissu.

3. Dispositif selon une des revendications 1 et 2, **caractérisé en ce que** le boitier (8) est muni à son extrémité ouverte d'une jupe (7) délimitant ladite surface d'étude et flexible pour limiter les variations de pression dues à l'application du dispositif contre ledit tissu par un utilisateur.

4. Dispositif selon la revendication 3, **caractérisé en ce qu'**il est prévu que soit interchangeable (ou soient interchangeables), de type matériel à usage unique désinfecté ou stérile, au moins un des éléments parmi ladite jupe (7) et/ou l'extrémité d'un piston (3) destinée à être en contact avec le tissu et comporté par les moyens de compression (3, 6).

5. Dispositif selon la revendication 4, **caractérisé en ce que** lesdits moyens de compression (3, 6) sont montés sur un ressort de rappel (61) actionné à la fin dudit premier temps déterminé pour ramener ledit piston (3) et annuler ladite pression contrôlée sur ladite zone (10).

6. Dispositif selon une des revendications 1 à 5, **caractérisé en ce que** lesdits moyens de compression (3, 6) comportent des moyens de contrepression (30) permettant de limiter les variations de pression dues à l'application du dispositif contre ledit tissu par un utilisateur.

7. Dispositif selon une des revendications 1 à 6, **caractérisé en ce que** lesdits moyens de compression (3, 6) comportent plusieurs pistons (3) appliquant, soit la même pression contrôlée, soit des pressions contrôlées différentes, sur plusieurs zones (10) dans ladite surface d'étude.

8. Dispositif selon une des revendications 1 à 6, **caractérisé en ce que** lesdits moyens de compression (3, 6) comportent un seul piston (3) munis de plusieurs saillies appliquant la même pression contrôlée sur plusieurs zones (10) dans ladite surface d'étude.

9. Dispositif selon une des revendications 7 et 8, **caractérisé en ce que** les moyens de traitement de données (800, 90) calculent au moins un paramètre relatif à la décoloration et/ou la recoloration dudit tissu sur chacune desdites zones (10) et comparent ce paramètre calculé entre lesdites zones (10) pour calculer au moins un paramètre relatif à l'homogénéité entre les paramètres calculés sur les différentes zones (10).

10. Dispositif selon une des revendications 1 à 9, **caractérisé en ce qu'**il comporte, à l'extrémité ouverte, au moins un capteur (70) de contact avec ledit tissu pour déclencher la mesure des premier et second temps déterminés.

11. Dispositif selon une des revendications 1 à 10, **caractérisé en ce qu'**il comporte des moyens de chauffage de ladite surface d'étude.

12. Système d'évaluation de la microcirculation sanguine d'un tissu humain ou animal, en particulier une muqueuse ou la peau, **caractérisé en ce qu'**il comporte au moins un dispositif selon l'une des revendications 1 à 11 coopérant avec au moins un dispositif informatique (9) comprenant des moyens de traitement de données (90) calculant ledit paramètre relatif à la décoloration et/ou la recoloration dudit tissu.

13. Système selon la revendication 12, **caractérisé en ce que** ledit dispositif informatique (9) comporte une interface homme-machine (100) fournissant à un utilisateur du dispositif ledit paramètre relatif à la décoloration et/ou la recoloration dudit tissu.

14. Système selon une des revendications 12 et 13, **caractérisé en ce que** ledit dispositif informatique (9) pilote des moyens de contrôle (80) du dispositif et utilise un premier temps déterminé et un second temps déterminé, pour contrôler l'application de ladite pression contrôlée sur ladite zone (10) pendant un premier temps déterminé, puis le retrait de ladite pression contrôlée et l'acquisition d'image sur ladite surface d'étude exposée à ladite lumière pendant un second temps déterminé.

15. Procédé d'utilisation d'un dispositif selon l'une des revendications 1 à 11 ou d'un système selon l'une des revendications 12 à 14, pour l'évaluation de la microcirculation sanguine d'un tissu humain ou animal, en particulier une muqueuse ou la peau, le procédé comprenant les étapes suivantes :
- Application dudit dispositif sur ledit tissu ;
- Application d'une pression calibrée sur ledit tissu pendant un premier temps déterminé, par les moyens de compression (3, 6) situés à l'extrémité ouverte dudit dispositif ;
- Illumination dudit tissu par une lumière à l'extrémité ouverte dudit dispositif et acquisition d'images dudit tissu par ledit dispositif, pendant un second temps déterminé ;
- Retrait des moyens de compression (3, 6) en dehors du champ d'acquisition d'images ;
- Calcul d'au moins un paramètre relatif à la décoloration et/ou la recoloration dudit tissu suite au retrait de ladite pression calibrée.

16. Procédé selon la revendication 15, **caractérisé en ce qu'**il comporte une analyse des images acquises par le dispositif, cette analyse comprenant les étapes suivantes :
- mesure d'une coloration de référence du tissu avant application de ladite pression calibrée ;
- calcul de la différence de la coloration du tissu après application de ladite pression calibrée par rapport à la coloration de référence, à plusieurs temps successifs après le retrait de ladite pression calibrée ;
- détermination d'un temps de recoloration, par mesure du temps auquel ladite différence de coloration redevient inférieure à un seuil déterminé.

## Patentansprüche

1. Vorrichtung für eine Auswertung der Blutmikrozirkulation eines menschlichen oder tierischen Gewebes, insbesondere einer Schleimhaut oder der Haut, die aufweist:
- ein Gehäuse (8), umfassend einen offenen Endpunkt (82), der dafür bestimmt ist, mit dem Gewebe in Kontakt gebracht zu werden und so eine Untersuchungsoberfläche des Gewebes zu begrenzen, die Vorrichtung umfassend:
- Mittel (2), zum Emittieren von Licht in Richtung der Untersuchungsoberfläche,
- Mittel (1) zum Erfassen von Bildern der Untersuchungsoberfläche,
- Mittel (3, 6) zum Komprimieren von mindestens einem bestimmten Gebiet (10) innerhalb der Untersuchungsoberfläche, wobei die Kompressionsmittel (3, 6) zum Anwenden eines kontrollierten Drucks auf das Gebiet (10) kalibriert sind und zum Zugänglichlassen der Kompressions- und/oder Untersuchungsoberfläche für das Licht und die Bilderfassungsmittel außerhalb des Bilderfassungsbereichs einklappbar sind,
- Kontrollmittel (80, 9), die die Anwendung des kontrollierten Drucks auf das Gebiet (10) während eines ersten bestimmten Zeitpunkts, dann die Aufhebung des kontrollierten Drucks und die Erfassung eines Bilds auf der Untersuchungsoberfläche, die dem Licht während eines zweiten bestimmten Zeitpunkts ausgesetzt wird, steuern,
- Datenverarbeitungsmittel (800, 90), die die erfassten Bilder verwenden, um mindestens einen Parameter zu berechnen, der sich auf die Verfärbung und/oder die Neufärbung des Gewebes bezieht.

2. Vorrichtung nach Anspruch 1, **dadurch gekennzeichnet, dass** der Parameter ein Zeitpunkt der Neufärbung des Gewebes ist.

3. Vorrichtung nach einem der Ansprüche 1 und 2, **dadurch gekennzeichnet, dass** das Gehäuse (8) an seinem offenen Endpunkt mit einer Schürze (7) versehen ist, die die Untersuchungsoberfläche begrenzt und flexibel ist, um die Druckschwankungen aufgrund der Anwendung der Vorrichtung gegen das Gewebe durch einen Benutzer einzugrenzen.

4. Vorrichtung nach Anspruch 3, **dadurch gekennzeichnet, dass** vorgesehen ist, dass mindestens eines der Elemente unter der Schürze (7) und/oder dem Endpunkt eines Kolbens (3), der dafür bestimmt ist, mit dem Gewebe in Kontakt zu sein und durch die Kompressionsmittel (3, 6) aufgewiesen wird, einer Art aus desinfiziertem oder sterilem Einwegmaterial, auswechselbar wäre (oder auswechselbar wären).

5. Vorrichtung nach Anspruch 4, **dadurch gekennzeichnet, dass** die Kompressionsmittel (3, 6) auf einer Rückstellfeder (61) montiert sind, die an dem Ende des ersten bestimmten Zeitpunkts betätigt wird, um den Kolben (3) zurückzubringen und den kontrollierten Druck auf das Gebiet (10) wegzunehmen.

6. Vorrichtung nach einem der Ansprüche 1 bis 5, **dadurch gekennzeichnet, dass** die Kompressionsmittel (3, 6) Gegendruckmittel (30) aufweisen, die es ermöglichen, die Druckschwankungen aufgrund der Anwendung der Vorrichtung gegen das Gewebe durch einen Benutzer einzugrenzen.

7. Vorrichtung nach einem der Ansprüche 1 bis 6, **dadurch gekennzeichnet, dass** die Kompressionsmittel (3, 6) mehrere Kolben (3) aufweisen, die entweder den gleichen kontrollierten Druck oder unterschiedliche kontrollierte Drücke auf mehrere Gebiete (10) in der Untersuchungsoberfläche anwenden.

8. Vorrichtung nach einem der Ansprüche 1 bis 6, **dadurch gekennzeichnet, dass** die Kompressionsmittel (3, 6) einen einzelnen Kolben (3) aufweisen, der mit mehreren Vorsprüngen versehen ist, die den gleichen kontrollierten Druck auf mehrere Gebiete (10) in der Untersuchungsoberfläche anwenden.

9. Vorrichtung nach einem der Ansprüche 7 und 8, **dadurch gekennzeichnet, dass** die Datenverarbeitungsmittel (800, 90) mindestens einen Parameter berechnen, der sich auf die Verfärbung und/oder die Neufärbung des Gewebes auf jedem der Gebiete (10) bezieht und diesen berechneten Parameter zwischen den Gebieten (10) vergleichen, um mindestens einen Parameter zu berechnen, der sich auf die Homogenität zwischen den berechneten Parametern auf den unterschiedlichen Gebieten (10) bezieht.

10. Vorrichtung nach einem der Ansprüche 1 bis 9, **dadurch gekennzeichnet, dass** sie an dem offenen Endpunkt mindestens einen Sensor (70) für den Kontakt mit dem Gewebe aufweist, um die Messung des ersten und des zweiten bestimmten Zeitpunkts auszulösen.

11. Vorrichtung nach einem der Ansprüche 1 bis 10, **dadurch gekennzeichnet, dass** sie Mittel zum Erwärmen der Untersuchungsoberfläche aufweist.

12. System für die Auswertung der Blutmikrozirkulation eines menschlichen oder tierischen Gewebes, insbesondere einer Schleimhaut oder der Haut, **dadurch gekennzeichnet, dass** es mindestens eine Vorrichtung nach einem der Ansprüche 1 bis 11 aufweist, die mit mindestens einer Computervorrichtung (9) zusammenwirkt, umfassend Datenverarbeitungsmittel (90), die den Parameter berechnen, der sich auf die Verfärbung und/oder die Neufärbung des Gewebes bezieht.

13. System nach Anspruch 12, **dadurch gekennzeichnet, dass** die Computervorrichtung (9) eine Mensch-Maschine-Schnittstelle (100) aufweist, die einem Benutzer der Vorrichtung den Parameter bereitstellt, der sich auf die Verfärbung und/oder die Neufärbung des Gewebes bezieht.

14. System nach einem der Ansprüche 12 und 13, **dadurch gekennzeichnet, dass** die Computervorrichtung (9) Kontrollmittel (80) der Vorrichtung steuert und einen ersten bestimmten Zeitpunkt und einen zweiten bestimmten Zeitpunkt verwendet, um die Anwendung des kontrollierten Drucks auf das Gebiet (10) während eines ersten bestimmten Zeitpunkts, dann die Aufhebung des kontrollierten Drucks und die Erfassung des Bilds auf der Untersuchungsoberfläche, die dem Licht während eines zweiten bestimmten Zeitpunkts ausgesetzt ist, zu steuern.

15. Verfahren zum Verwenden einer Vorrichtung nach einem der Ansprüche 1 bis 11 oder eines Systems nach einem der Ansprüche 12 bis 14 zum Auswerten der Blutmikrozirkulation eines menschlichen oder tierischen Gewebes, insbesondere einer Schleimhaut oder der Haut, das Verfahren umfassend die folgenden Schritte:
- Anwenden der Vorrichtung auf das Gewebe;
- Anwenden eines kalibrierten Drucks auf das Gewebe während eines ersten bestimmten Zeitpunkts durch die Kompressionsmittel (3, 6), die an dem offenen Endpunkt der Vorrichtung gelegen sind;
- Beleuchten des Gewebes durch ein Licht an dem offenen Endpunkt der Vorrichtung und Erfassen von Bildern des Gewebes durch die Vorrichtung während eines zweiten bestimmten Zeitpunkts;
- Aufheben der Kompressionsmittel (3, 6) außerhalb des Bilderfassungsbereichs;
- Berechnen mindestens eines Parameters, der sich auf die Verfärbung und/oder die Neufärbung des Gewebes nach dem Aufheben des kalibrierten Drucks bezieht.

16. Verfahren nach Anspruch 15, **dadurch gekennzeichnet, dass** es eine Analyse der Bilder aufweist, die durch die Vorrichtung erfasst werden, diese Analyse umfassend die folgenden Schritte:
- Messen einer Referenzfärbung des Gewebes vor der Anwendung des kalibrierten Drucks;
- Berechnen des Unterschieds der Färbung des Gewebes nach der Anwendung des kalibrierten Drucks relativ zu der Referenzfärbung zu mehreren aufeinanderfolgenden Zeitpunkten nach dem Aufheben des kalibrierten Drucks;
- Bestimmen eines Neufärbungszeitpunkts durch Messen des Zeitpunkts, zu dem der Färbungsunterschied kleiner als ein bestimmter Schwellenwert ist.

## Claims

1. Device for evaluating the blood microcirculation of human or animal tissue, in particular a mucous membrane or the skin, comprising:
- a housing (8) comprising an open end (82), intended to be placed in contact with said tissue and thus delimit a study surface of said tissue, the device comprising:
- light emitting means (2) towards the study surface,
- image acquisition means (1) of the study surface,
- compression means (3, 6) of at least one determined zone (10) inside the study surface, said compression means (3, 6) being calibrated to apply a controlled pressure on said zone (10) and retractable outside the image acquisition field to leave said compression and/or study surface accessible to said light and to the image acquisition means,
- control means (80, 9) controlling the application of said controlled pressure on said area (10) for a first determined time, then the withdrawal of said controlled pressure and the image acquisition on said exposed study surface to said light for a second determined time,
- data processing means (800, 90) using the acquired images to calculate at least one parameter relating to the discoloration and/or recoloration of said tissue.

2. Device according to claim 1, **characterized in that** the said parameter is a recoloration time of the said tissue.

3. Device according to one of claims 1 and 2, **characterized in that** the housing (8) is provided at its open end with a skirt (7) delimiting the said study surface and flexible to limit the pressure variations due to the application of the device against said tissue by a user.

4. Device according to claim 3, **characterized in that** at least one of the elements among the said skirt (7) and/or the end of a piston (3) intended to be in contact with the tissue and comprised by the compression means (3, 6) is (or are) interchangeable, of the disinfected or sterile single-use material type.

5. Device according to claim 4, **characterized in that** the said compression means (3, 6) are mounted on a return spring (61) actuated at the end of the said first determined time to return the said piston (3) and cancel the said controlled pressure on said area (10).

6. Device according to one of claims 1 to 5, **characterized in that** the said compression means (3, 6) comprise counterpressure means (30) making it possible to limit the pressure variations due to the application of the device against the said tissue by a user.

7. Device according to one of claims 1 to 6, **characterized in that** the said compression means (3, 6) comprise several pistons (3) applying either the same controlled pressure or different controlled pressures to several zones (10) in the said study area.

8. Device according to one of claims 1 to 6, **characterized in that** the said compression means (3, 6) comprise a single piston (3) provided with several projections applying the same controlled pressure to several zones (10) in the said study surface.

9. Device according to one of claims 7 and 8, **characterized in that** the data processing mean (800, 90) calculate at least one parameter relating to the discoloration and/or the recoloration of the said tissue on each of the said zones (10) and compare this parameter calculated between said zones (10) to calculate at least one parameter relating to the homogeneity between the parameters calculated on the different zones (10).

10. Device according to one of claims 1 to 9, **characterized in that** it comprises, at the open end, at least one sensor (70) of contact with the said tissue to trigger the measurement of the first and second determined times.

11. Device according to one of claims 1 to 10, **characterized in that** it comprises means for heating the said study surface.

12. System for evaluating the blood microcirculation of human or animal tissue, in particular a mucous membrane or the skin, **characterized in that** it comprises at least one device according to one of claims 1 to 11 cooperating with at least one electronic device (9) comprising data processing means (90) calculating said parameter relating to the discoloration and/or recoloration of said tissue.

13. System according to claim 12, **characterized in that** the said electronic device (9) includes a man-machine interface (100) providing a user of the device with the said parameter relating to the discoloration and/or the recoloration of the said tissue.

14. System according to one of claims 12 and 13, **characterized in that** the said electronic device (9) controls control means (80) of the device and uses a first determined time and a second determined time to control the application of the said controlled pressure on said area (10) for a first determined time, then withdrawing said controlled pressure and acquiring an image on said study surface exposed to said light for a second determined time.

15. Method of using a device according to one of claims 1 to 11 or a system according to one of claims 12 to 14, for the evaluation of the blood microcirculation of human or animal tissue, in particular a mucous membrane or the skin, the method comprising the following steps:
- Application of said device to said tissue;
- Application of a calibrated pressure on said tissue for a first determined time, by the compression means (3 6) located at the open end of said device;
- Illumination of said tissue by a light at the open end of said device and acquisition of images of said tissue by said device, for a second determined time;
- Removal of the compression means (3, 6) outside the image acquisition field;
- Calculation of at least one parameter relating to the discoloration and/or recoloration of said tissue following the removal of said calibrated pressure.

16. Method according to claim 15, **characterized in that** it includes an analysis of the images acquired by the device, this analysis comprising the following steps:
- measurement of a reference coloration of the tissue before applying said calibrated pressure;
- calculation of the difference in the coloration of the tissue after application of said calibrated pressure compared to the reference coloration, at several successive times after the removal of said calibrated pressure;
- determination of a recoloration time, by measuring the time at which said color difference becomes less than a determined threshold.
